# FASCICULE DE BREVET EUROPEEN

(11) **EP 1 474 369 B1**
(45) Date de publication et mention de la délivrance du brevet: **24.11.2010**
(21) Numéro de dépôt: 03720616.6
(22) Date de dépôt: 17.02.2003
(51) Int. Cl.: C07B 63/04, C07C 39/08, C07C 37/88

(54) **PROCEDE DE PREPARATION D'UNE COMPOSITION LIQUIDE D'UN COMPOSE BENZENIQUE ORTHO-DIHYDROXYLE DE HAUTE PURETE ET COMPOSITION OBTENUE.**
VERFAHREN ZUR HERSTELLUNG EINER FLÜSSIGEN ZUSAMMENSETZUNG VON EINER SEHR REINEN ORTHO-DIHYDROXY-BENZOL-VERBINDUNG SOWIE DIE ERHALTENE ZUSAMMENSETZUNG
METHOD FOR PRODUCTION OF A LIQUID COMPOSITION OF AN ORTHO-DIHYDROXYBENZYL COMPOUND OF HIGH PURITY AND SAID COMPOSITION

(30) Priorité: 15.02.2002 FR 0201925
(43) Date de publication de la demande: 10.11.2004
(73) Titulaire: RHODIA CHIMIE, 92512 Boulogne Billancourt Cedex (FR)
(72) Inventeur: MATHIEU, Claude, F-69960 Corbas (FR)
(74) Mandataire: Dutruc-Rosset, Marie-Claude
(86) Numéro de dépôt international: PCT/FR2003/000497
(87) Numéro de publication internationale: WO 2003/068713

(56) Documents cités:
- GB-A- 1 347 533

## Description

La présente invention a pour objet un procédé de préparation d'une composition liquide d'un composé benzénique ortho-dihydroxylé de haute pureté et la composition ainsi obtenue.

L'invention concerne plus particulièrement la préparation d'une composition liquide de pyrocatéchol de haute pureté.

Certains domaines d'application, par exemple l'électronique requièrent des produits d'une très grande pureté notamment au niveau des éléments métalliques. Ainsi, le pyrocatéchol ou 1,2-dihydroxybenzène est introduit dans des formulations destinées à enlever les polymères « photoresists » qui sont utilisés pour la gravure des circuits intégrés et imprimés. Il est important de ne pas polluer le semi-conducteur par des éléments métalliques qui risqueraient de créer des perturbations.

Le pyrocatéchol, en raison de ses propriétés complexantes, est également utilisé comme chélatants et comme inhibiteurs de corrosion.

Il existe donc sur le marché une forte demande de produits extrêmement purs.

Il n'est pas aisé d'obtenir de tels produits à l'échelle industrielle. En effet, en raison de leurs propriétés chélantes, l'obtention de tels produits exempts d'impuretés est très difficile.

De plus, le pyrocatéchol étant un produit disponible sur le marché sous forme de poudre ou d'écailles est connu pour son irritabilité et tous les utilisateurs doivent prendre des précautions en terme d'hygiène industrielle pour le manipuler. A partir d'un produit sous forme solide, les utilisateurs devront assurer des manipulations, des mises en solution dans des industries ne disposant pas toujours des équipements bien adaptés.

Un produit plus pur est généralement obtenu suite à des opérations de distillation et de mise en forme (crisatllisation, broyage, écaillage, ...) afin d'obtenir une forme solide manipulable du pyrocatéchol. Toutefois, il devient très vite pollué à sortie de la colonne de distillation dès qu'il est en contact avec des matériaux que l'on rencontre dans les dispositifs de mise en forme du fait des proprités abrasives du pyrocatéchol.

Ainsi, il est possible de rencontrer les éléments chimiques suivants : Al, Sb, As, Ba, Be, Bi, B, Cd, CI, Ca, Cr, Co, Cu, Ga, Au, Fe, Li, Mg, Mn, Ni, K, Pb, Ag, Na, Sr, Ta, TI, Sn, Ti, V, Zn, Zr, Hg, I.

Certains éléments métalliques en quantités prépondérantes sont : le fer, l'aluminium, le chrome, le magnésium, le nickel, le calcium, le manganèse, le sodium et le potassium.

Pour pallier cet inconvénient, il a été proposé selon EP-A 1 055 657 de produire un composé benzénique dihydroxylé ayant une teneur totale en métal de moins de 200 ppb qui consiste à évaporer ledit composé dans une colonne à distillation, à condenser la fraction vapeur dudit composé dans un condenseur relié à une sortie et à récupérer la fraction condensée dans un bac de stockage par l'intermédiaire d'un conduit relié au condenseur, lequel condenseur a une surface interne constituée par un matériau métallique comprenant au moins 25 % en poids de nickel et la fraction condensée vient en contact avec ladite surface.

Il est à noter que les exemples relatifs au pyrocatéchol mettent en évidence une différence de pureté du produit obtenu soit 15 ppb par rapport à l'utilisation d'un dispositif classique qui conduit à l'obtention d'un produit ayant une teneur totale en impuretés métalliques de 592 ppb. Cependant, le texte est muet sur la récupération du pyrocatéchol à la sortie du condenseur.

L'objet de la présente invention est de fournir non pas le pyrocatéchol sous forme solide mais sous la forme d'une composition liquide de pyrocatéchol de haute pureté aisément mise en oeuvre par les utilisateurs, sans risque de pollution subséquente.

L'invention vise à fournir un composé benzénique ortho-dihydroxylé de haute pureté sous forme liquide à température ambiante.

Par « température ambiante », on entend généralement une température comprise dans une gamme de températures allant de 0°C à 35°C, de préférence comprise entre 15°C et 25°C.

Par «haute pureté », on entend une pureté chimique du composé benzénique ortho-dihydroxylé de départ déterminée par chromatographie en phase gazeuse d'au moins 99 % et une teneur de chaque élément métallique d'au plus 100 ppb, de préférence inférieure à 60 ppb.

Dans le présent texte, la teneur en élément métallique est défini par rapport au composé benzénique ortho-dihydroxylé et non pas par rapport à la solution.

Par « liquide », on inclut selon l'invention, les produits visqueux mais toujours à l'état liquide.

L'un des objets de l'invention est constitué par les compositions liquides d'un composé benzénique ortho-dihydroxylé de haute pureté.

Plus précisément, l'invention consiste en une composition liquide d'un composé benzénique ortho-dihydroxylé de haute pureté caractérisée par le fait qu'elle comprend au moins un composé benzénique ortho-dihydroxylé de haute pureté et au moins un solvant organique solubilisant ledit composé mis en oeuvre en une quantité suffisante pour obtenir une composition liquide.

Un autre objet de l'invention est le procédé de préparation de ladite composition liquide caractérisé par le fait qu'il consiste :
- à purifier par distillation le composé benzénique ortho-dihydroxylé de départ et à récupérer le composé benzénique ortho-dihydroxylé condensé dans un appareillage résistant à la corrosion de ce dernier,
- à effectuer ensuite son mélange avec un solvant organique dans un bac de stockage revêtu ou constitué d'un matériau n'apportant pas de pollution métallique.

La présente invention vise à fournir des compositions liquides d'un composé benzénique ortho-dihydroxylé de haute pureté comprenant au moins un composé benzénique ortho-dihydroxylé et au moins un solvant organique.

De manière préférée, les compositions de l'invention comprennent au moins un ou plusieurs composés benzéniques ortho-dihydroxylés de formule (I) : 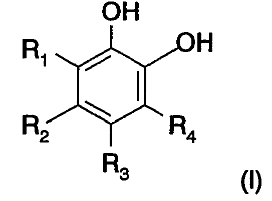
- dans ladite formule :
   - R₁, R₂, R₃, et R₄, identiques ou différents, représentent un atome d'hydrogène, un groupe alkyle, un groupe alkoxy, un atome d'halogène, un groupe trifluorométhyle, un groupe nitro ou un groupe carboxylique COOH, un groupe CHO,
   - au moins un substituant parmi R₁, R₂, R₃, et R₄ représente un atome d'hydrogène,
   - au plus deux substituants parmi R₁, R₂, R₃, et R₄ représentent un atome d'halogène, un groupe trifluorométhyle, un groupe nitro ou un groupe carboxylique COOH, un groupe CHO.

Par « alkyle », on entend selon l'invention une chaîne hydrocarbonée linéaire ou ramifiée ou cyclique ayant de 1 à 12 atomes de carbone, telle que par exemple, méthyle, éthyle, n-propyle, isopropyle, n-butyle, sec-butyle, tert-butyle et octyle. Les groupes alkyle C₁-C₄ sont préférés.

Par « alkoxy », on entend selon l'invention un groupe alkyl-oxy où le groupe alkyle est tel que défini précédemment. De préférence, le groupe alkoxy est un groupe isopropoxy.

Par « halogène », on entend l'atome de fluor, de chlore ou de brome.

Un groupe de composés benzéniques ortho-dihydroxylés préférés est constitué par des composés de formule (I) dans lesquels au moins trois substituants parmi R₁, R₂, R₃, et R₄ représentent un atome d'hydrogène. Parmi ceux-ci, ceux pour lesquels le quatrième substituant R₁, R₂, R₃ ou R₄ est choisi parmi un atome d'hydrogène, un groupe alkyle C₁-C₁₂, de préférence, C₁-C₄ et un groupe alkoxy en C₁-C₁₂, de préférence en C₁-C₄, sont encore plus avantageux.

Un groupe de composés préférés est celui constitué le pyrocatéchol, le 3-méthylpyrocatéchol, le 4-méthylpyrocatéchol, le 3-isopropylpyrocatéchol, le 3-butyl-5-méthylpyrocatéchol, le 4-tert-butylpyrocatéchol, le 2-chloropyrocatéchol, le 4-chloropyrocatéchol, le 3,5-di-tert-butylpyrocatéchol, le 4,6-di-tert-butylpyrocatéchol, le 3-octyl-5-méthylpyrocatéchol, le 4-isopropoxypyrocatéchol, le 3,6-diisopropylpyrocatéchol, le 4-nitropyrocatéchol, l'aldéhyde protocatéchique.

Parmi les composés précités, le pyrocatéchol est le composé préféré.

On notera que tous les différents composés benzéniques ortho-dihydroxylés sont commercialisés ou facilement préparés par l'Homme du Métier.

Pour ce qui est du solvant organique, son choix est fait en tenant compte de plusieurs impératifs.

Ce solvant doit être inerte vis-à-vis du composé benzénique ortho-dihydroxylé et être susceptible de le solubiliser.

Il est également choisi eu égard l'utilisation envisagée.

On peut mettre en oeuvre un mélange de solvants organiques.

On fait appel préférentiellement à un solvant organique polaire.

Comme exemples spécifiques, on peut citer notamment :
- les composés de type azotés éventuellement hydroxylés tels que : éthylène diamine, NMP (N-méthylpyrrrolidone), pyridine, MEA (monoéthanolamine), diéthanolamine, triéthanolamine, tert-butyldiéthanolamine, isopropanolamine, 2-amino-1-propanolamine, 3-amino-1-propanolamine, isobutanolamine, 2-amino-2-éthoxyéthanol, DGA [diglycolamine ou 2-(2-aminoéthoxy)éthanol],
- les composés de type alcool et/ou éther et/ou ester tels que : éthylène glycol, propylène glycol ; TEG [triéthylène glycol], glyme, diglyme, PGMEA [propylène glycol monométhyl éther acétate ou acétate de 2-(1-méthoxy)propyle], PGME [propylèneglycol monométhyléther], lactate d'éthyle, anisole, adipate de méthyle, cyclopentanol,
- les composés de type hydrocarbure tels que toluène, xylène, mésitylène,
- les composés de type cétone tels que méthyléthylcétone, 2-pentanone, cyclopentanone, cyclohexanone, oxyde de mésityle,
- le diméthylsulfoxyde.

Afin d'obtenir une composition liquide, on met en oeuvre préférentiellement :
- de 5 à 80 % en poids d'un composé benzénique ortho-dihydroxylé,
- de 20 à 95 % en poids d'un solvant organique.

Les compositions préférées de l'invention comprennent :
- de 5 à 50 % en poids d'un composé benzénique ortho-dihydroxylé,
- de 50 à 95 % en poids d'un solvant organique.

L'invention s'applique tout à fait bien à la préparation de compositions liquides de pyrocatéchol de haute pureté.

Elles comprennent préférentiellement de 5 à 50 %, de préférence de 15 à 40 % de pyrocatéchol et de 50 à 95 %, de préférence de 60 à 85 % d'un solvant organique.

Les compositions préférées selon l'invention comprennent de 5 à 50 % en poids de pyrocatéchol et de 50 à 95 % en poids de DGA ou MEA.

Les compositions liquides de pyrocatéchol obtenues présentent la pureté définie ci-dessus.

Avantageusement, le total de tous les éléments métalliques est inférieur à 100 ppb, de préférence inférieur à 80 ppb.

Conformément à l'invention, on obtient une composition liquide d'un composé benzénique ortho-dihydroxylé avec une haute pureté en élément métallique en mettant en oeuvre le procédé de l'invention.

Plus précisément, on part d'un composé benzénique ortho-dihydroxylé généralement disponible sur le marché à une bonne pureté le plus souvent supérieure à 50 %.

On effectue sa purification par distillation.

Il est à noter que ledit composé peut être en mélange avec d'autres composés. Tel est le cas pour l'hydroquinone et le pyrocatéchol qui sont produits simultanément par hydroxylation du phénol par le peroxyde d'hydrogène, en présence d'un acide fort généralement l'acide perchlorique ou l'acide sulfurique.

La distillation est conduite à une température comprise entre 120°C et 200°C sous une pression allant de 30 à 100 mm de mercure.

Elle est effectuée dans un appareil de distillation classique.

L'homme du métier est parfaitement en mesure de choisir les moyens à mettre en oeuvre en fonction du composé benzénique ortho-dihydroxylé de départ.

On rappellera simplement ce qui suit. La taille (notamment le diamètre) des colonnes de distillation dépend du flux circulant et de la pression interne. Leur dimensionnement se fera donc principalement suivant le débit de mélange à traiter. Le paramètre interne qu'est le nombre d'étages théoriques est déterminé notamment par la pureté du composé de départ et la pureté du produit devant être obtenus en tête de distillation.

On précisera que les colonnes pourront être garnies indifféremment de plateaux ou de garnissage ordonné, comme cela est parfaitement connu de l'homme du métier.

L'installation étant déterminée, l'homme du métier ajuste les paramètres de fonctionnement de la colonne.

Ainsi, la colonne de distillation pourra être avantageusement, mais non limitativement, une colonne ayant les spécifications suivantes :
- nombre d'étages théoriques : de 1 à 50, de préférence de 20 à 30,
- taux de reflux R compris entre 0,5 et 10 , de préférence entre 3 et 6.

En bas de colonne, on récupère les produits non désirés, en particulier les produits organiques de point d'ébullition plus élevé contenant la totalité des traces métalliques.

En tête de colonne, on récupère une phase gazeuse contituée par le composé benzénique ortho-dihydroxylé purifié.

On refroidit la phase gazeuse et la transforme sous forme liquide par refroidissement à une température légèrement supérieure à son point de critallisation, par exemple supérieure de 5 à 20°C, de préférence supérieure à 10°C.

Cette opération est conduite par passage dans un condenseur qui est un appareil classique par exemple un échangeur tubulaire alimenté par de l'eau ou par un fluide (généralement une huile) maintenu à une température voisine de la température de refroidissement choisie.

Conformément à une caractéristique du procédé de l'invention, il importe que le haut de la colonne de distillation et le condenseur n'apportent pas de pollution métallique.

A cet effet, on choisit avantageusement les aciers inoxydables, tels que les aciers austénitiques et plus particulièrement les aciers inoxydables 304, 304 L, 316 ou 316 L.

On met en oeuvre un acier ayant au plus 22 % en poids de nickel, le plus souvent compris entre 6 et 20 %, de préférence compris entre 8 et 14 %.

Les aciers 304 et 304 L ont une teneur en nickel variant entre 8 et 12 % et les aciers 316 et 316 L ont une teneur en nickel variant entre 10 et 14 %.

De tels aciers sont utilisés couramment dans l'industrie.

Pour la définition des aciers austénitiques, on peut se référer à l'ouvrage de Robert H. Perry et al, [Perry's Chemical Engineers' Handbook, Sixth Edition (1984), page 23-44].

On récupère le composé benzénique ortho-dihydroxylé le plus près de son point de distillation et on le mélange le plus rapidement possible avec un solvant organique exempt d'éléments métalliques.

A cet effet, on véhicule le produit purifié par l'intermédiaire de tuyaux en acier inoxydable tel que précédement défini ou en matière plastique puis l'on effectue le mélange avec le solvant organique dans un bac de stockage en acier inoxydable ou en matière plastique.

Il est possible d'envisager un ou plusieurs bacs de stockage. Dans ce dernier cas, le produit peut être véhiculé par l'intermédiaire de pompes en acier inxoydable.

Il est à noter que le temps de séjour du composé benzénique ortho-dihydroxylé étant très court dans ces tuyauterie, les matériaux devront être résistants à la corrosion mais aucune exigence mécanique ne leur est demandée.

Dans le cas où le matériau utilisé est l'acier inoxydable, il y a lieu auparavant de rincer un ou plusieurs (3 ou 4) fois, l'outil concerné avec le composé benzénique ortho-dihydroxylé que l'on souhaite obtenir en solution.

Il y a lieu également de noter que tout stockage prolongé est avantageusement effectué dans des containers en matière plastique.

Comme matières plastiques, on choisit un composé polymérique résistant à la corrosion de la composition de l'invention. On peut citer notamment, les matériaux tels que PTFE (polytétrafluoroéthylène ou Teflon) ou PFA (résines perfluoroalkyles), polyéthylène haute densité. On ne sortira pas du cadre de l'invention à utiliser un matériau équivalent.

D'une manière préférée, le composé benzénique ortho-dihydroxylé purifié est avantageusement véhiculé par l'intermédiaire d'un tuyau en matière plastique ou comprenant un revêtement en matière plastique dans un bac de stockage en matière plastique dans lequel est effectué le mélange avec le solvant organique.

On récupère ainsi directement la composition liquide du composé benzénique ortho-dihydroxylé de haute pureté.

Par rapport aux autres solutions préconisées dans la littérature, la présentation sous forme liquide a l'avantage de supprimer la partie mise en forme solide qui nécessite des matériaux chers à haute teneur en nickel et des équipements de conditionnement et de dépotage pour solide.

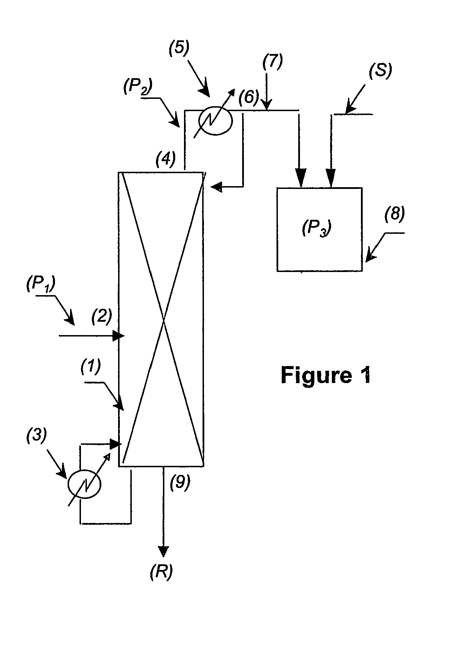

On a représenté sur la figure 1 annexée un mode de réalisation préférée de l'invention.

L'installation permettant la préparation des compositions de l'invention comprend une première colonne (1) équipée des moyens d'admission (2) du composé benzénique ortho-dihydroxylé à purifier, sous forme liquide, (*P₁*) envoyé préférentiellement par l'intermédiaire d'une pompe dans la colonne, généralement en milieu de colonne.

La colonne est munie dans sa partie inférieure de moyens de chauffage [bouilleur] (3) et dans sa partie supérieure de moyens d'évacuation (4) de la phase gazeuse (*P₂*) qui traverse un condenseur (5) et à sa sortie (6), une fraction déterminée par le taux de reflux est renvoyée dans la colonne et l'autre fraction est envoyée par l'intermédiaire d'un conduit en acide inoxydable, ou de préférence en matière plastique (7) dans un bac de stockage (8) en acier inoxydable ou en matière plastique dans lequel est ajouté le solvant organique (S) par gravité, de préférence sans passage par l'intermédiaire de pompe.

On peut charger au préalable le solvant (S) dans (8) avant l'addition du composé benzénique ortho-dihydroxylé.

On récupère ainsi directement la composition liquide d'un composé benzénique ortho-dihydroxylé de haute pureté dans le bac (8) et en bas de colonne (9), les produits non désirés.

On donne ci-après des exemples de réalisation de l'invention et des exemples comparatifs.

### Exemple 1

On se réfère à la figure (1) pour la description de l'exemple.

Dans une colonne à distiller (1) garnie, ayant un diamètre d'approximativement de 1,0 mètre et une hauteur d'environ de 6 mètres en acier inoxydable 304 L, on introduit en (2) à un débit de 500 kg/h, un mélange d'hydroquinone et de pyrocatéchol comprenant le pyrocatéchol de départ a un degré de pureté d'environ 45 % en poids et contenant plus de 100 ppb de fer.

On chauffe par l'intermédiaire d'un bouilleur (3) situé en pied de colonne à une température de l'ordre de 210°C.

Le taux de reflux de la colonne est ajusté pour obtenir le taux de pureté souhaité.

De la vapeur est fournie au bouilleur (3) à un débit qui est ajusté en fonction du taux de reflux.

Il est choisi de l'ordre de 3.

En pied de colonne (9), on récupère les produits non désirés à savoir l'hydroquinone et les éléments métalliques.

La phase gazeuse récupérée en tête de colonne en (4) est envoyée dans un condenseur (5). Elle est condensée à une température de 120°C.

Une partie du flux condensée au sortie du condenseur (6) est renvoyée dans la colonne selon le taux de reflux et l'autre partie est envoyée dans un container en PFA (8) par l'intermédiaire d'un tuyau en PFA (7).

Le pyrocatéchol sortant du condenseur et entrant dans le container contenant le solvant a une pureté plus grande que 99,5 % en poids et contient les éléments suivants : Sb ; As ; Ba ; Be ; Bi ; B ; Cd ; Cr ; Co ; Cu ; Ga ; Au ; Li ; Mn ; Ni ; Pb ; Ag ; Sr ; Ta ; Tl ; Sn ; V ; Zn ; Zr ; Hg ; I ; chacun des éléments sont à moins de 0,1 ppb
Al : 5 ppb
Ca : 7,4 ppb
Fe : 15 ppb
Mg : 2,8 ppb
K : 7,9 ppb
Na : 35 ppb
Ti : 0,3 ppb
ou un total de moins de 80 ppb des 33 composés analysés.

L'analyse a été faite par une des méthodes les plus performantes ICP/MS (Induction Couple Plasma / Mass Spectrometry).

Le solvant à savoir le DGA est introduit au préalable dans le container (8) à raison de 800 kg, le pyrocatéchol étant ensuite récupéré jusqu'à obtention d'un volume de 200 kg ce qui correspond à l'obtention d'une solution de pyrocatéchol à 20 % en poids.

### Exemple 2 :

Au bout de 1000 heures de fonctionnement conduit selon l'exemple 1, on obtient un pyrocatéchol ayant la pureté suivante : les éléments suivants à moins de 0,1 ppb chacun : I ; Sb ; As ; Ba ; Be ; Bi ; B ; Cd ; Co ; Cu ; Ga ; Au ; Li ; Mn ; Ni;Pb;Ag;Sr;Ta;Tl,Sn;Ti;V;Zn;Zr;Hg;
K : 12 ppb
Mg : 2 ppb
Ca : 12ppb
Fe : 6 ppb
Na : 26 ppb
Cr : 24 ppb

Le total des 33 éléments est de moins de 85 ppb.

### Exemple comparatif 3

On part d'un pyrocatéchol purifié. La teneur de chaque métal est inférieure à 50 ppb.

On soumet le pyrocatéchol distillé à un écaillage sur écailleuse à tambour, couteau en bronze, tambour en inox.

Le pyrocatéchol écaillé obtenu a une teneur en fer supérieure à 100 ppb.

### Exemple 4

On prépare une solution de pyrocatéchol à 21,5 % en poids dans la monoéthanolamine MEA.

A cet effet, on répète l'exemple 1 mais en mettant en oeuvre la monoéthanolamine.

Le pyrocatéchol à la sortie du condenseur, a une pureté plus grande que 99,5 % en poids et contient les éléments suivants :
Al : 2 ppb
Fe : 13 ppb
Cr : 15 ppb
Ca : 5 ppb
Mg : 2 ppb
K : 5 ppb
Na : 8 ppb
Bi : 5 ppb
Pb : 2 ppb

Les autres éléments sont à moins de 0,1 ppb.

### Exemple 5

Des résultats similaires à l'exemple 1 sont obtenus en mettant en oeuvre du pyrocatéchol purifié par distillation sur colonne à garnissage ordonné en acier inoxydable (Sulzer BX) ayant un diamètre de 1 mètre. La phase gazeuse récupérée en tête de colonne est envoyée vers un condenseur d'une surface d'échange de 75 m².

Le pyrocatéchol sortant du condenseur traverse un premier bac de stockage en acier inoxydable (304), une pompe en acier inoxydable, un tuyau en acier inoydable (316 L) de 200 m, un deuxième bac de stockage en acier inoxydable (316 L), une pompe en acier inoxydable pour le transférer dans un bac de stockage en matière plastique (polyéthylène haute densité) contenant le solvant.

Il est à noter que dans le but d'obtenir la solution de pyrocatéchol haute pureté, il y a lieu au préalable de rinçer l'installation avec du pyrocatéchol pur.

Le pyrocatéchol avant d'être mélangé avec le solvant DGA a une pureté plus grande que 99,5 % en poids et contient les éléments suivants : Sb ; As ; Ba; Be ; Bi; B ; Cd ; Cr ; Co ; Cu ; Ga ; Au ; Li ; Mn ; Ni; Pb ; Ag ; Sr ; Ta ; TI; Sn ; V ; Zn ; Zr ; Hg ; I ; chacun des éléments sont à moins de 0,1 ppb
Al : 4,1 ppb
Ca : 4,3 ppb
Fe : 13 ppb
Mg : 1,5 ppb
K : 3,9 ppb
Na : 69 ppb
Ti : 1 ppb
ou un total de moins de 100 ppb des 33 composés analysés.

On obtient une solution de pyrocatéchol à 20 % en poids dans la DGA.

## Revendications

1. Procédé de préparation d'une composition liquide d'un composé benzénique ortho-dihydroxylé de haute pureté **caractérisé par le fait qu'**il consiste :
- à purifier par distillation le composé benzénique ortho-dihydroxylé de départ et à récupérer le composé benzénique ortho-dihydroxylé condensé dans un appareillage résistant à la corrosion de ce dernier,
- à effectuer ensuite son mélange avec un solvant organique dans un bac de stockage revêtu ou constitué d'un matériau n'apportant pas de pollution métallique.

2. Procédé selon la revendication 2 **caractérisé par le fait que** le composé benzénique ortho-dihydroxylé est prélevé le plus près de son point de distillation.

3. Procédé selon l'une des revendications 2 et 3 **caractérisé par le fait que** l'on effectue la distillation et la condensation du composé benzénique ortho-dihydroxylé purifié dans un appareillage en acier austénitique, de préférence en acier inoxydable 304, 304 L, 316 ou 316 L.

4. Procédé selon la revendication 3 **caractérisé par le fait que** l'acier a au plus 22 % en poids de nickel, de préférence de 6 à 20 %, et encore plus préférentiellement de 8 à 14 %.

5. Procédé selon l'une des revendications 1 à 4 **caractérisé par le fait que** l'on véhicule le composé benzénique ortho-dihydroxylé purifié par l'intermédiaire d'un tuyau en acier inoxydable défini dans la revendication précédente ou un tuyau en matière plastique ou comprenant un revêtement en matière plastique.

6. Procédé selon l'une des revendications 1 à 5 **caractérisé par le fait que** l'on effectue le mélange du composé benzénique ortho-dihydroxylé purifié et du solvant organique dans un bac de stockage en acier inoxydable ou en matière plastique.

7. Procédé selon l'une des revendications 1 à 6 **caractérisé par le fait que** l'on effectue la distillation et la condensation du composé benzénique ortho-dihydroxylé purifié dans un appareillage en acier inoxydable, que l'on véhicule le composé benzénique ortho-dihydroxylé purifié par l'intermédiaire d'un tuyau en acier inoxydable ou d'un tuyau en matière plastique ou comprenant un revêtement en matière plastique et que l'on effectue le mélange du composé benzénique ortho-dihydroxylé purifié et du solvant organique dans un bac de stockage en acier inoxydable ou en matière plastique.

8. Procédé selon l'une des revendications 1 à 7 **caractérisé par le fait que** l'on soumet l'outil en acier inoxydable à un ou plusieurs rinçages effectués à l'aide du composé benzénique ortho-dihydroxylé que l'on souhaite mettre en solution.

9. Procédé selon l'une des revendications 1, 5 à 7, **caractérisé par le fait que** la matière plastique est le PTFE (polytétrafluoroéthylène ou Teflon) ou PFA (résines perfluoroalkyles), le polyéthylène haute densité.

10. Procédé selon l'une des revendications 1 à 9 **caractérisé par le fait que** le composé benzénique ortho-dihydroxylé répond à la formule (I) : 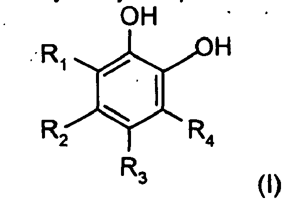
- dans ladite formule :
. R₁, R₂, R₃, et R₄, identiques ou différents, représentent un atome d'hydrogène, un groupe alkyle, un groupe alkoxy, un atome d'halogène, un groupe trifluorométhyle, un groupe nitro ou un groupe carboxylique COOH, un groupe CHO,
au moins un substituant parmi R₁, R₂, R₃, et R₄ représente un atome d'hydrogène,
au plus deux substituants parmi R₁, R₂, R₃, et R₄ représentent un atome d'halogène, un groupe trifluorométhyle, un groupe nitro ou un groupe carboxylique COOH, un groupe CHO.

11. Procédé selon la revendication 10 **caractérisé par le fait que** le composé benzénique ortho-dihydroxylé répond à la formule (I) dans laquelle au moins trois substituants parmi R₁, R₂, R₃, et R₄ représentent un atome d'hydrogène ; le quatrième substituant R₁, R₂, R₃, ou R₄, étant choisi parmi un atome d'hydrogène, un groupe alkyle C₁-C₁₂, de préférence, C₁-C₄ et un groupe alkoxy en C₁-C₁₂, de préférence en C₁-C₄.

12. Procédé selon la revendication 10 **caractérisé par le fait que** le composé benzénique ortho-dihydroxylé est choisi parmi : le pyrocatéchol, le 3-méthylpyrocatéchol, le 4-méthylpyrocatéchol, le 3-isopropylpyrocatéchol, le 3-butyl-5-méthylpyrocatéchol, le 4-tert-butylpyrocatéchol, le 2-chloropyrocatéchol, le 4-chloropyrocatéchol, le 3,5-di-tert-butylpyrocatéchol, le 4,6-di-tert-butylpyrocatéchol, le 3-octyl-5-méthylpyrocatéchol, le 4-isopropoxypyrocatéchol, le 3,6-diisopropylpyrocatéchol, le 4-nitropyrocatéchol, l'aldéhyde protocatéchique.

13. Procédé selon l'une des revendications 1 à 12 **caractérisé par le fait que** le solvant organique est choisi parmi :
- les composés de type azotés éventuellement hydroxylés tels que : éthylène diamine, NMP (N-méthylpyrrrolidone), pyridine, MEA (monoéthanolamine), diéthanolamine, triéthanolamine, tert-butyldiéthanolamine, isopropanolamine, 2-amino-1-propanolamine, 3-amino-1-propanolamine, isobutanolamine, 2-amino-2-éthoxyéthanol, DGA [diglycolamine ou 2-(2-aminoéthoxy)éthanol],
- les composés de type alcool et/ou éther et/ou ester tels que : éthylène glycol, propylène glycol ; TEG [triéthylène glycol], glyme, diglyme, PGMEA [propylène glycol monométhyl éther acétate ou acétate de. 2-(1-méthoxy)propyle], PGME [propylèneglycol monométhyléther], lactate d'éthyle, anisole, adipate de méthyle, cyclopentanol,
- les composés de type hydrocarbure tels que toluène, xylène, mésitylène,
- les composés de type cétone tels que méthyléthylcétone, 2-pentanone, cyclopentanone, cyclohexanone, oxyde de mésityle,
- le diméthylsulfoxyde.

14. Procédé selon la revendication 1 à 13 **caractérisé par le fait que** la composition liquide comprend de 5 à 80 %, de préférence de 5 à 50 % en poids d'un composé benzénique ortho-dihydroxylé et de 20 à 95 %, de préférence, de 50 à 95 % en poids d'un solvant organique.

15. Procédé selon la revendication 1 à 14 **caractérisé par le fait que** la composition liquide comprend de 5 à 50 % en poids de pyrocatéchol de haute pureté et de 50 à 95 % en poids de DGA (diglycolamine) ou MEA (monoéthanolamime).

16. Composition liquide d'un composé benzénique ortho-dihydroxylé de haute pureté comprenant ledit composé et un solvant organique dudit composé obtenue selon le procédé décrit dans l'une des revendications 1 à 15.

17. Composition liquide de pyrocatéchol comprenant :
- de 5 à 80 % en poids de pyrocatéchol et de 20 à 95 % en poids d'un solvant organique, de préférence de 5 à 50 % en poids de pyrocatéchol et de 50 à 95 % en poids d'un solvant organique,
- une teneur de chaque élément métallique d'au plus 100 ppb, de préférence inférieure à 60 ppb.

18. Composition liquide de pyrocatéchol selon la revendication 17 comprenant :
- de 5 à 80 % en poids de pyrocatéchol et de 20 à 95 % en poids d'un solvant organique, de préférence de 5 à 50 % en poids de pyrocatéchol et de 50 à 95 % en poids d'un solvant organique,
- la teneur de tous les éléments métalliques inférieur à 100 ppb, de préférence inférieur à 80 ppb.

19. Composition selon l'une des revendications 17 et 18 **caractérisée par le fait que** le solvant organique est choisi parmi :
- les composés de type azotés éventuellement hydroxylés tels que : éthylène diamine, NMP (N-méthylpyrrrolidone), pyridine, MEA (monoéthanolamine), diéthanolamine, triéthanolamine, tert-butyldiéthanolamine, isopropanolamine, 2-amino-1-propanolamine, 3-amino-1-propanolamine, isobutanolamine, 2-amino-2-éthoxyéthanol, DGA [diglycolamine ou 2-(2-aminoéthoxy)éthanol],
- les composés de type alcool et/ou éther et/ou ester tels que : éthylène glycol, propylène glycol ; TEG [triéthylène glycol], glyme, diglyme, PGMEA [propylène glycol monométhyl éther acétate ou acétate de 2-(1-méthoxy)propyle], PGME [propylèneglycol monométhyléther], lactate d'éthyle, anisole, adipate de méthyle, cyclopentanol,
- les composés de type hydrocarbure tels que toluène, xylène, mésitylène,
- les composés de type cétone tels que méthyléthylcétone, 2-pentanone, cyclopentanone, cyclohexanone, oxyde de mésityle,
- le diméthylsulfoxyde.

20. Composition selon l'une des revendications 17 et 18, **caractérisée par le fait qu'**elle comprend :
- de 5 à 50 % en poids de pyrocatéchol de haute pureté,
- de 50 à 95 % en poids de DGA (diglycolamine), ou MEA (monoéthanolamine).

## Claims

1. Method for production of a liquid composition of an ortho-dihydroxybenzyl compound of high purity, **characterized in that** it consists:
- in purifying the starting ortho-dihydroxybenzyl compound by distillation and in recovering the condensed ortho-dihydroxybenzyl compound in an apparatus resistant to corrosion by the latter,
- in subsequently mixing said condensed ortho-dihydroxybenzyl compound with an organic solvent in a storage tank coated with or constituted of a material that does not introduce any metal pollution.

2. Method according to Claim 2, **characterized in that** the ortho-dihydroxybenzyl compound is taken as close to its distillation point as possible.

3. Method according to either of Claims 2 and 3, **characterized in that** the distillation and the condensation of the purified ortho-dihydroxybenzyl compound are carried out in an apparatus made of austenitic steel, preferably made of stainless steel 304, 304 L, 316 or 316 L.

4. Method according to Claim 3, **characterized in that** the steel has at most 22% by weight of nickel, preferably from 6% to 20%, and even more preferably from 8% to 14%.

5. Method according to one of Claims 1 to 4, **characterized in that** the purified ortho-dihydroxybenzyl compound is conveyed by means of a pipe made of stainless steel defined in the preceding claim or a pipe made of plastic or comprising a plastic coating.

6. Method according to one of Claims 1 to 5, **characterized in that** the mixing of the purified ortho-dihydroxybenzyl compound and the organic solvent is carried out in a storage tank made of stainless steel or of plastic.

7. Method according to one of Claims 1 to 6, **characterized in that** the distillation and the condensation of the purified ortho-dihydroxybenzyl compound are carried out in a stainless steel apparatus, **in that** the purified ortho-dihydroxybenzyl compound is conveyed by means of a pipe made of stainless steel or a pipe made of plastic or comprising a plastic coating and **in that** the mixing of the purified ortho-dihydroxybenzyl compound and the organic solvent is carried out in a storage tank made of stainless steel or of plastic.

8. Method according to one of Claims 1 to 7, **characterized in that** the stainless steel tool is subjected to one or more rinses performed using the ortho-dihydroxybenzyl compound that it is desired to put into solution.

9. Method according to one of Claims 1 and 5 to 7, **characterized in that** the plastic is PTFE (polytetrafluoroethylene or Teflon) or PFA (perfluoroalkyl resins), or high density polyethylene.

10. Method according to one of Claims 1 to 9, **characterized in that** the ortho-dihydroxybenzyl compound corresponds to formula (I): 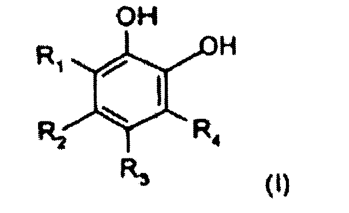
- in which formula:
• R₁, R₂, R₃ and R₄, which may be identical or different, represent a hydrogen atom, an alkyl group, an alkoxy group, a halogen atom, a trifluoromethyl group, a nitro group, a carboxylic group COOH or a CHO group,
• at least one substituent among R₁, R₂, R₃ and R₄ represents a hydrogen atom,
• at most two substituents among R₁, R₂, R₃ and R₄ represent a halogen atom, a trifluoromethyl group, a nitro group, a carboxylic group COOH or a CHO group.

11. Method according to Claim 10, **characterized in that** the ortho-dihydroxybenzyl compound corresponds to formula (I) in which at least three substituents among R₁, R₂, R₃ and R₄ represent a hydrogen atom, the fourth substituent R₁, R₂, R₃ or R₄ being chosen from a hydrogen atom, a C₁-C₁₂, preferably C₁-C₄, alkyl group and a C₁-C₁₂, preferably C₁-C₄, alkoxy group.

12. Process according to Claim 10, **characterized in that** the ortho-dihydroxybenzyl compound is chosen from: pyrocatechol, 3-methylpyrocatechol, 4-methylpyrocatechol, 3-isopropylpyrocatechol, 3-butyl-5-methylpyrocatechol, 4-tert-butylpyrocatechol, 2-chloropyrocatechol, 4-chloropyrocatechol, 3,5-di-tert-butylpyrocatechol, 4,6-di-tert-butylpyrocatechol, 3-octyl-5-methylpyrocatechol, 4-isopropoxypyrocatechol, 3,6-diisopropylpyrocatechol, 4-nitropyrocatechol and protocatechualdehyde.

13. Method according to one of Claims 1 to 12, **characterized in that** the organic solvent is chosen from:
- compounds of optionally hydroxylated nitrogenous type, such as: ethylenediamine, NMP (N-methylpyrrolidone), pyridine, MEA (monoethanolamine), diethanolamine, triethanolamine, tert-butyldiethanolamine, isopropanolamine, 2-amino-1-propanolamine, 3-amino-1-propanolamine, isobutanolamine, 2-amino-2-ethoxyethanol or DGA [diglycolamine or 2-(2-aminoethoxy)ethanol],
- compounds of alcohol and/or ether and/or ester type, such as: ethylene glycol, propylene glycol; TEG [triethylene glycol], glyme, diglyme, PGMEA [propylene glycol monomethyl ether acetate or 2-(1-methoxy)propyl acetate], PGME [propylene glycol monomethyl ether], ethyl lactate, anisole, methyl adipate or cyclopentanol,
- compounds of hydrocarbon type, such as toluene, xylene or mesitylene,
- compounds of ketone type, such as methyl ethyl ketone, 2-pentanone, cyclopentanone, cyclohexanone or mesityl oxide,
- dimethyl sulphoxide.

14. Method according to Claims 1 to 13, **characterized in that** the liquid composition comprises from 5% to 80%, preferably from 5% to 50%, by weight, of an ortho-dihydroxybenzyl compound and from 20% to 95%, preferably from 50% to 95%, by weight, of an organic solvent.

15. Method according to Claims 1 to 14, **characterized in that** the liquid composition comprises from 5% to 50% by weight of pyrocatechol of high purity and from 50% to 95% by weight of DGA (diglycolamine) or MEA (monoethanolamine).

16. Liquid composition of an ortho-dihydroxybenzyl compound of high purity, which comprises said compound and an organic solvent for said compound and which is obtained according to the method described in one of Claims 1 to 15.

17. Liquid composition of pyrocatechol, comprising:
- from 5% to 80% by weight of pyrocatechol and from 20% to 95% by weight of an organic solvent, preferably from 5% to 50% by weight of pyrocatechol and from 50% to 95% by weight of an organic solvent,
- a content of each metal element of at most 100 ppb, preferably less than 60 ppb.

18. Liquid composition of pyrocatechol according to Claim 17, comprising:
- from 5% to 80% by weight of pyrocatechol and from 20% to 95% by weight of an organic solvent, preferably from 5% to 50% by weight of pyrocatechol and from 50% to 95% by weight of an organic solvent,
- a content of all the metal elements of less than 100 ppb, preferably less than 80 ppb.

19. Composition according to either of Claims 17 and 18, **characterized in that** the organic solvent is chosen from:
- compounds of optionally hydroxylated nitrogenous type, such as: ethylenediamine, NMP (N-methylpyrrolidone), pyridine, MEA (monoethanolamine), diethanolamine, triethanolamine, tert-butyldiethanolamine, isopropanolamine, 2-amino-1-propanolamine, 3-amino-1-propanolamine, isobutanolamine, 2-amino-2-ethoxyethanol or DGA [diglycolamine or 2-(2-aminoethoxy)ethanol],
- compounds of alcohol and/or ether and/or ester type, such as: ethylene glycol, propylene glycol; TEG [triethylene glycol], glyme, diglyme, PGMEA [propylene glycol monomethyl ether acetate or 2-(1-methoxy)propyl acetate], PGME [propylene glycol monomethyl ether], ethyl lactate, anisole, methyl adipate or cyclopentanol,
- compounds of hydrocarbon type, such as toluene, xylene or mesitylene,
- compounds of ketone type, such as methyl ethyl ketone, 2-pentanone, cyclopentanone, cyclohexanone or mesityl oxide,
- dimethyl sulphoxide.

20. Composition according to either of Claims 17 and 18, **characterized in that** it comprises:
- from 5% to 50% by weight of pyrocatechol of high purity,
- from 50% to 95% by weight of DGA (diglycolamine) or MEA (monoethanolamine).

## Patentansprüche

1. Verfahren zur Herstellung einer flüssigen Zusammensetzung einer ortho-Dihydroxybenzolverbindung hoher Reinheit, **dadurch gekennzeichnet, daß** man:
- die als Ausgangsstoff dienende ortho-Dihydroxybenzolverbindung destillativ reinigt und die kondensierte ortho-Dihydroxybenzolverbindung in einer Apparatur, die gegenüber Korrosion durch letztere beständig ist, zurückgewinnt und
- sie dann in einem Speicherbehälter, der mit einem Material überzogen ist oder aus einem Material besteht, das keine Metallverunreinigung mit sich bringt, mit einem organischen Lösungsmittel mischt.

2. Verfahren nach Anspruch 1, **dadurch gekennzeichnet, daß** man die ortho-Dihydroxybenzolverbindung am nächsten an ihrem Destillationspunkt abzieht.

3. Verfahren nach einem der Ansprüche 1 und 2, **dadurch gekennzeichnet, daß** man die Destillation und die Kondensation der gereinigten ortho-Dihydroxybenzolverbindung in einer Apparatur aus austenitischem Stahl, vorzugsweise nichtrostendem Stahl 304, 304 L, 316 oder 316 L, durchführt.

4. Verfahren nach Anspruch 3, **dadurch gekennzeichnet, daß** der Stahl höchstens 22 Gew.-% Nickel, vorzugsweise 6 bis 20% und noch weiter bevorzugt 8 bis 14%, aufweist.

5. Verfahren nach einem der Ansprüche 1 bis 4, **dadurch gekennzeichnet, daß** man die gereinigte ortho-Dihydroxybenzolverbindung mit Hilfe eines Rohrs aus nichtrostendem Stahl gemäß der im vorhergehenden Anspruch angegebenen Definition oder eines Rohrs aus Kunststoff oder eines Rohrs mit einem Kunststoffüberzug befördert.

6. Verfahren nach einem der Ansprüche 1 bis 5, **dadurch gekennzeichnet, daß** man das Mischen der gereinigten ortho-Dihydroxybenzolverbindung und des organischen Lösungsmittels in einem Speicherbehälter aus nichtrostendem Stahl oder Kunststoff durchführt.

7. Verfahren nach einem der Ansprüche 1 bis 6, **dadurch gekennzeichnet, daß** man die Destillation und die Kondensation der gereinigten ortho-Dihydroxybenzolverbindung in einer Apparatur aus austenitischem Stahl durchführt, die gereinigte ortho-Dihydroxybenzolverbindung mit Hilfe eines Rohrs aus nichtrostendem Stahl oder eines Rohrs aus Kunststoff oder eines Rohrs mit einem Kunststoffüberzug befördert und das Mischen der gereinigten ortho-Dihydroxybenzolverbindung und des organischen Lösungsmittels in einem Speicherbehälter aus nichtrostendem Stahl oder Kunststoff durchführt.

8. Verfahren nach einem der Ansprüche 1 bis 7, **dadurch gekennzeichnet, daß** man das Gerät aus nichtrostendem Stahl ein- oder mehrmals mit der ortho-Dihydroxybenzolverbindung, die in Lösung gebracht werden soll, wäscht.

9. Verfahren nach einem der Ansprüche 1 und 5 bis 7, **dadurch gekennzeichnet, daß** es sich bei dem Kunststoff um PTFE (Polytetrafluorethylen oder Teflon), PFA (Perfluoralkylharze) oder Polyethylen hoher Dichte handelt.

10. Verfahren nach einem der Ansprüche 1 bis 9, **dadurch gekennzeichnet, daß** die ortho-Dihydroxybenzolverbindung der Formel (I) entspricht: 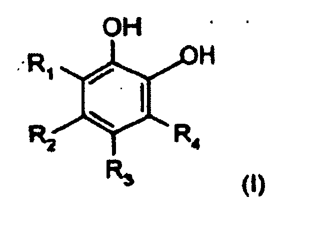
- worin:
• R₁, R₂, R₃ und R₄ gleich oder verschieden sind und für ein Wasserstoffatom, eine Alkylgruppe, eine Alkoxygruppe, ein Halogenatom, eine Trifluormethylgruppe, eine Nitrogruppe, eine Carboxylgruppe COOH oder eine CHO-Gruppe stehen,
mindestens einer der Substituenten R₁, R₂, R₃ und R₄ für ein Wasserstoffatom steht,
• höchstens zwei der Substituenten R₁, R₂, R₃ und R₄ für ein Halogenatom, eine Trifluormethylgruppe, eine Nitrogruppe, eine Carboxylgruppe COOH oder eine CHO-Gruppe stehen.

11. Verfahren nach Anspruch 10, **dadurch gekennzeichnet, daß** die ortho-Dihydroxybenzolverbindung der Formel (I) entspricht, worin mindestens drei der Substituenten R₁, R₂, R₃ und R₄ für ein Wasserstoffatom stehen, wobei der vierte Substituent R₁, R₂, R₃ oder R₄ unter einem Wasserstoffatom, einer C₁-C₁₂-Alkylgruppe, vorzugsweise einer C₁-C₄-Alkylgruppe, und einer C₁-C₁₂-Alkoxygruppe, vorzugsweise einer C₁-C₄-Alkoxygruppe, ausgewählt wird.

12. Verfahren nach Anspruch 10, **dadurch gekennzeichnet, daß** die ortho-Dihydroxybenzolverbindung unter Pyrocatechol, 3-Methylpyrocatechol, 4-Methylpyrocatechol, 3-Isopropylpyrocatechol, 3-Butyl-5-methylpyrocatechol, 4-tert.-Butylpyrocatechol, 2-Chlorpyrocatechol, 4-Chlorpyrocatechol, 3,5-Di-tert.-butylpyrocatechol, 4,6-Di-tert.-butylpyrocatechol, 3-Octyl-5-methylpyrocatechol, 4-Isopropoxypyrocatechol, 3,6-Diisopropylpyrocatechol, 4-Nitropyrocatechol und Protocatechualdehyd ausgewählt wird.

13. Verfahren nach einem der Ansprüche 1 bis 12, **dadurch gekennzeichnet, daß** das organische Lösungsmittel unter
- gegebenenfalls hydroxylierten Stickstoffverbindungen wie Ethylendiamin, NMP (N-Methylpyrrolidon), Pyridin, MEA (Monoethanolamin), Diethanolamin, Triethanolamin, tert.-Butyl-diethanolamin, Isopropanolamin, 2-Amino-1-propanolamin, 3-Amino-1-propanolamin, Isobutanolamin, 2-Amino-2-ethoxyethanol oder DGA [Diglykolamin oder 2-(2-Aminoethoxy)ethanol],
- Alkohol- und/oder Ether- und/oder Esterverbindungen wie Ethylenglykol, Propylenglykol, TEG [Triethylenglykol], Glyme, Diglyme, PGMEA [Propylenglykolmonomethyletheracetat oder Essigsäure-2-(1-methoxy)propylester], PGME [Propylenglykolmonomethylether], Milchsäureethylester, Anisol, Adipinsäuremethylester oder Cyclopentanol,
- Kohlenwasserstoffverbindungen wie Toluol, Xylol, Mesitylen,
- Ketonverbindungen wie Methylethylketon, 2-Pentanon, Cyclopentanon, Cyclohexanon oder Mesityloxid,
- Dimethylsulfoxid
ausgewählt wird.

14. Verfahren nach Anspruch 1 bis 13, **dadurch gekennzeichnet, daß** die flüssige Zusammensetzung 5 bis 80 Gew.-%, vorzugsweise 5 bis 50 Gew.-%, einer ortho-Dihydroxybenzolverbindung und 20 bis 95 Gew.-%, vorzugsweise 50 bis 95 Gew.-%, eines organischen Lösungsmittels umfaßt.

15. Verfahren nach Anspruch 1 bis 14, **dadurch gekennzeichnet, daß** die flüssige Zusammensetzung 5 bis 50 Gew.-% Pyrocatechol hoher Reinheit und 50 bis 95 Gew.-% DGA (Diglykolamin) oder MEA (Monoethanolamin) umfaßt.

16. Flüssige Zusammensetzung einer ortho-Dihydroxybenzolverbindung hoher Reinheit, umfassend die Verbindung und ein organisches Lösungsmittel für die Verbindung und erhalten nach dem in einem der Ansprüche 1 bis 15 beschriebenen Verfahren.

17. Flüssige Pyrocatecholzusammensetzung, umfassend:
- 5 bis 80 Gew.-% Pyrocatechol und 20 bis 95 Gew.-% eines organischen Lösungsmittels, vorzugsweise 5 bis 50 Gew.-% Pyrocatechol und 50 bis 95 Gew.-% eines organischen Lösungsmittels,
- einen Gehalt jedes Metallelements von höchstens 100 ppb, vorzugsweise weniger als 60 ppb.

18. Flüssige Pyrocatecholzusammensetzung nach Anspruch 17, umfassend:
- 5 bis 80 Gew.-% Pyrocatechol und 20 bis 95 Gew.-% eines organischen Lösungsmittels, vorzugsweise 5 bis 50 Gew.-% Pyrocatechol und 50 bis 95 Gew.-% eines organischen Lösungsmittels,
- einen Gehalt aller Metallelemente von weniger als 100 ppb, vorzugsweise weniger als 80 ppb.

19. Zusammensetzung nach einem der Ansprüche 17 und 18, **dadurch gekennzeichnet, daß** das organische Lösungsmittel unter
- gegebenenfalls hydroxylierten Stickstoffverbindungen wie Ethylendiamin, NMP (N-Methylpyrrolidon), Pyridin, MEA (Monoethanolamin), Diethanolamin, Triethanolamin, tert.-Butyl-diethanolamin, Isopropanolamin, 2-Amino-1-propanolamin, 3-Amino-1-propanolamin, Isobutanolamin, 2-Amino-2-ethoxyethanol oder DGA [Diglykolamin oder 2-(2-Aminoethoxy)ethanol],
- Alkohol- und/oder Ether- und/oder Esterverbindungen wie Ethylenglykol, Propylenglykol, TEG [Triethylenglykol], Glyme, Diglyme, PGMEA [Propylenglykolmonomethyletheracetat oder Essigsäure-2-(1-methoxy)propylester], PGME [Propylenglykolmonomethylether], Milchsäureethylester, Anisol, Adipinsäuremethylester oder Cyclopentanol,
- Kohlenwasserstoffverbindungen wie Toluol, Xylol, Mesitylen,
- Ketonverbindungen wie Methylethylketon, 2-Pentanon, Cyclopentanon, Cyclohexanon oder Mesityloxid,
- Dimethylsulfoxid
ausgewählt wird.

20. Zusammensetzung nach einem der Ansprüche 17 und 18, **dadurch gekennzeichnet, daß** die flüssige Zusammensetzung
- 5 bis 50 Gew.-% Pyrocatechol hoher Reinheit und
- 50 bis 95 Gew.-% DGA (Diglykolamin) oder MEA (Monoethanolamin)
umfaßt.
